# EUROPEAN PATENT SPECIFICATION

(11) **EP 1 898 825 B1**
(45) Date of publication and mention of the grant of the patent: **20.07.2011**
(21) Application number: 06779479.2
(22) Date of filing: 20.09.2006
(51) Int. Cl.: A61B 18/20

(54) **LASER HAIR REMOVAL DEVICE**
VORRICHTUNG ZUR HAARENTFERNUNG MITTELS LASER
DISPOSITIF LASER D'ELIMINATION DE POIL

(30) Priority: 21.09.2005 GB 0519252
(43) Date of publication of application: 19.03.2008
(60) Divisional application: 10178043.5
(73) Proprietor: The Dezac Group Limited, Cheltenham Gloucestershire GL50 1SS (GB)
(72) Inventor: SOHI, Daniel, Essex IG11 8JX (GB); WOLSKI, Alexander, Gloucester GL4 4XG (GB); BITTER, Ahmad, London W2 6PP (GB); GRANT, Tony, Stroud GL5 4SG (GB)
(74) Representative: Newell, William Joseph
(86) International application number: PCT/GB2006/003470
(87) International publication number: WO 2007/034161

(56) References cited:
- EP-A- 1 238 683
- EP-A1- 1 285 600
- EP-A2- 0 913 127
- WO-A-98/07379
- WO-A-03/049633
- WO-A-2004/032665
- WO-A-2004/080279
- WO-A1-02/094116
- DE-A1- 1 541 165
- US-A- 4 617 926
- US-A- 5 820 625
- US-A1- 2004 167 500
- US-A1- 2004 167 502
- US-B1- 6 572 637

## Description

This invention relates to laser hair removal devices and in particular, but not exclusively, to such devices intended for home usage.

In the past, cosmetic hair removal has been achieved in numerous ways including plucking, electrolysis and laser treatment. In the laser treatment process, light at a suitable wavelength (typically from about 600nm to 900nm) is directed towards the skin such that some of the energy is absorbed by the papilla of the hair follicle, which is damaged by the deposition of energy leading to death of the follicle and subsequent hair removal.

Because of the complexity of these devices and the safety considerations inherent with their use, traditionally laser hair removal treatment has been carried out at specialist clinics or salons rather than at home.

There is therefore a need for a laser hair removal device with inbuilt safety features so that it can be safely used by the domestic market.

WO03/049633 discloses an arrangement in which a short flash is emitted prior to firing the laser beam. There is no disclosure of an arrangement in which a beam is turned on to deter the user from aiming the device towards their eye.

EP0913127 discloses a device for use by a specialist operator in which a light pointing device can selectively pass a guide ray (for aiming purposes) or an atrophying ray.

Accordingly, in an aspect, this invention concerns a hair removal device utilising a laser generating pulses of sufficient energy and duration to damage papilla of each hair follicle in the path of the beam, with one or more safety features designed to present accidental misuse of the device.

In one particular aspect of this invention, there is a laser hair removal device for being directed towards and/or applied to the skin of a user to expose hair follicles to laser radiation for hair removal, said device comprising: a body, a hair removal laser housed in said body and capable of emitting radiation along a main axis at a wavelength suitable for hair removal, a further light emitting element arranged to emit high intensity radiation selected to stimulate an eye blinking reflex and non-injurious to the eye, and a control circuit which, at or following power up of the device, and prior to actuation of said hair removal laser, turns on said further light emitting element to emit a beam of radiation, wherein, when turned on, said further light emitting element projects a high intensity beam of visible light along an axis in generally the same direction as said main axis thereby to provide a warning of the projected axis of the radiation from said hair removal laser and to deter a user from aiming the device towards their eye.

The high intensity beam is non-injurious in the sense that whilst it might be unpleasant to look into the beam, no permanent injury is caused by that beam.

Preferably the high intensity beam is turned on as soon as the device is powered. In most instances, the hair removal laser will be fired on demand using a trigger or the like. The laser will typically generate a pulsed beam of laser radiation. The light emitting element preferably emits light at a wavelength to which the eye is particularly sensitive such as light of wavelength of the order of 500-600nm and ideally around 555nm. The light emitting element may comprise an LED designed to provide light at the required frequency.

It should be appreciated that the various functions In the above aspects may be performed by components mounted in an applicator type device which contains at least the majority of the control functionality required for operation. Alternatively, the device may be in the form of a separate base unit which contains the majority of the control circuitry with a separate head containing the laser unit and connected to the base unit by a flexible link.

Whilst the invention has been described above, it extends to any inventive combination of the features set out above, or in the following description.

The invention may be performed in various ways, and two embodiments thereof will now be described by way of example only, reference being made to the accompanying drawings in which:
Figure 1 is a schematic diagram of a first embodiment of a laser hair removal device in accordance with this invention;
Figure 2 illustrates a second embodiment of the a hair removal device in accordance with the invention; and
Figures 3, 4 and 5 are views of a part of the hair removal device shown in Figure 2.

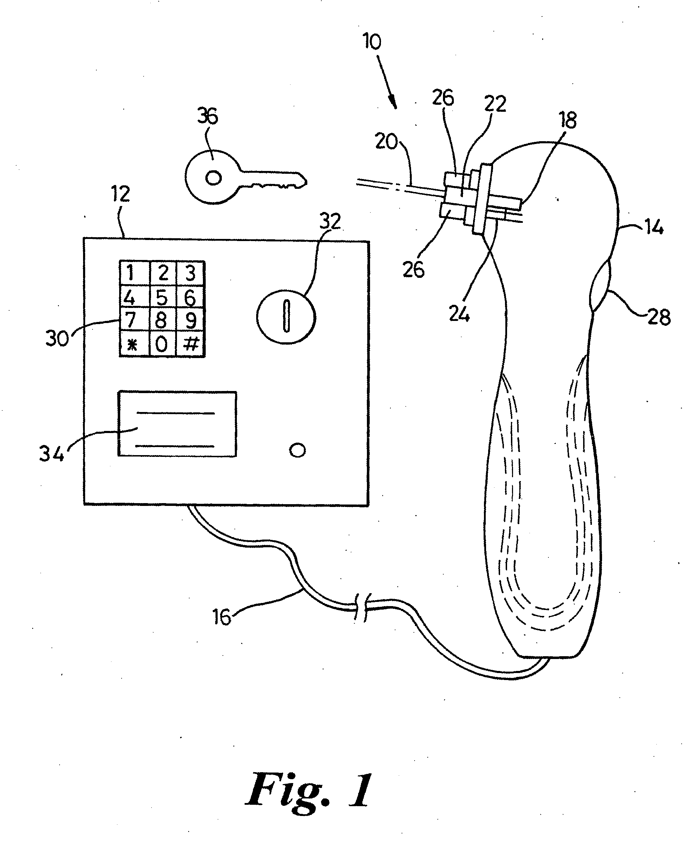

Referring to Figure 1, the hair removal device 10 comprises a base unit 12 and a body in the form of a laser wand 14 connected to the base unit by means of a retractable cord 16. In this particular embodiment the safety control circuitry is contained in the base unit 12 although in other embodiments all or part of this circuitry may be contained in the laser wand 14. The laser wand contains a laser 18 which produces a pulse beam of laser radiation at a wavelength selected to cause hair removal. The laser 18 is arranged to project its beam along a main axis 20. The laser 18 is housed inside the wand 14 and the beam, on leaving the main body of the wand 14 passes through a cylindrical shield sleeve 22 of semi-transparent material. Adjacent the laser 18 is a LED 24 designed also to project a high intensity beam of non-injurious visible light along substantially the same main axis 20, passing down the middle of the sleeve 22. To either side of the sleeve are provided two electrodes 26 which are connected to an electrical control circuit which detects when the laser wand 14 is in contact with the skin of a person. This is done by detecting the resistivity of the skin's surface contacted by the electrodes 26. It would be noted that, when the laser wand 14 is pressed against the skin with both contacts 26 in touch with the skin, the laser beam emitted from laser 18 is enclosed by the sleeve 22.

On the back of the wand is an operating switch 28 whose state is determined by the control circuitry in the base unit 12.

On the base unit there is an alphanumeric keypad 30 and a key operated switch 32 together with a display 34. In use, the control circuit in the base unit 12 inhibits operation of the laser 18 until certain conditions are fulfilled. In this particular example, it is necessary firstly to turn the unit on using a key 36 in the key switch 32 and then to punch in a PIN code through the keypad 30. When these steps have been carried out, the control unit activates the LED 24 so that a high intensity beam of visible light is projected along the main axis 20 both as a warning of the projected axis of the laser beam from laser 18 when it is activated and also to deter users from pointing it towards their own or others' eyes. Having passed the initial security steps, the wand 14 may be offered up to the area of the skin where hair is to be removed and contact with the skin (as opposed to a non-skin surface) is detected by the control circuit using the electrodes 26. Then, and only then, can the main laser 18 be activated by pressing the operating switch 28.

It will therefore be appreciated that the embodiment illustrated in Figure 1 requires several security stages to be passed before the laser can be operated; the first level security checks confirm the credentials of the user (i.e. being in possession of the right key and code) and thereafter physical security is assessed by ensuring that the device is actually in contact with the skin before the laser is enabled.

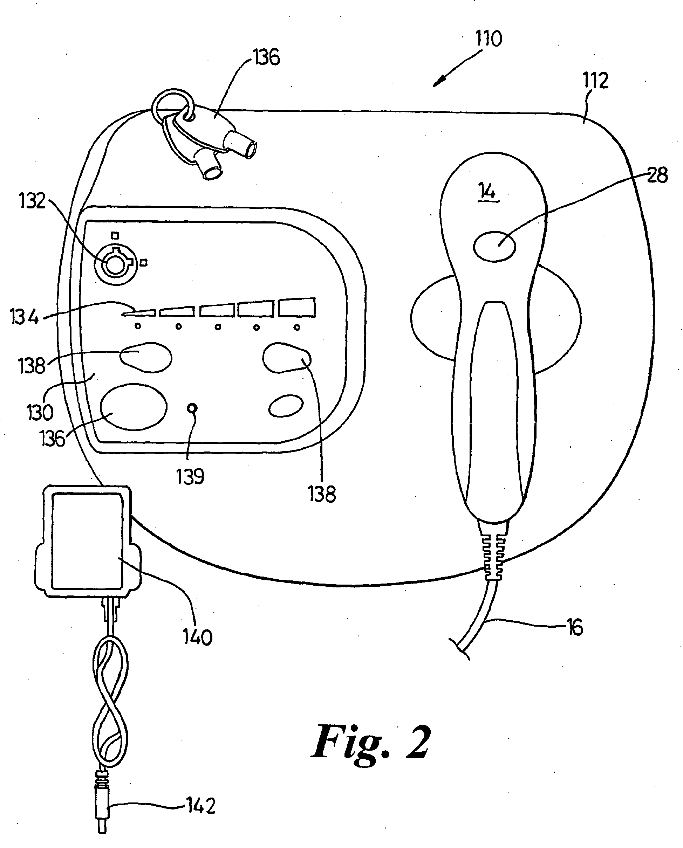

A second embodiment of a hair removal device is illustrated in Figures 2 to 5. Features common to the first and second embodiments have like numbering. The hair removal device 110 has a base unit 112 and a hand held laser wand 14 which operate in a similar manner to the base unit and wand 12,14 described above. In this embodiment the wand 14 rests on, and is held in place by the housing 112. The base unit has a mains adapter power supply 140 which has a low voltage d.c. output plug 142 for fitting into and powering the base unit 112. Alternatively, or as well as, the base unit 122 may be powered by batteries, which may be rechargeable via the mains adapter.

In operation the base unit can be switched on by inserting key 136 into keyswitch 132. As an additional safety precaution, a predetermined series of buttons on the keypad 130 may have to be pressed to enable the laser wand 14 to be powered. The base unit has a power level indicator 134, an enable/hold button 136 , power up/down button 138, and a wand enable warning light 139. The wand 14 has at least the same safety features described above.

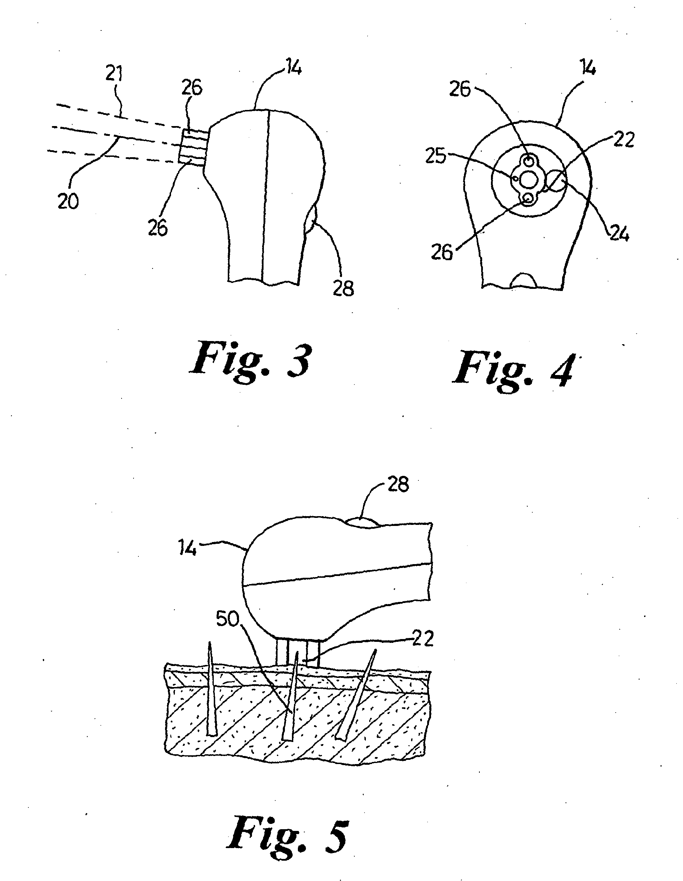

Figures 3,4 & 5 show different views of the head of the wand 14. In Figure 3, electrodes 26 are visible for making contact with, or coming into close proximity with, the skin of a user to enable the wand. If the laser were able to fire, the path of the coherent laser light would pass along axis 20. The beam 21 of high intensity visible but non-injurious light, which is diffuse light, travels along the generally the same axis 20. The diffuse beam 21 is of a lower intensity than the laser beam and is produced when the base 112 is powered or just before the laser operation in order to stimulate an eye blinking reflex of any eye in the path of the beam 21.

Figure 4 shows a different view of the head of the wand 14. In this embodiment there is shown a low intensity parallel beam LED 25 which produces a low intensity narrow beam of light along the axis 20. The purpose of this light is to aim the laser so as to guide the user to the hair to be removed. This light can be switched on and can remain on before the laser is operated. A further high intensity LED 24 produces the high intensity light beam 21 for promoting blinking and this light is produced just before the laser is fired.

Figure 5 shows the wand in use aimed, with the aid of the low intensity light from the LED 25. In this position the electrodes 26 are in contact with the skin of a user adjacent body hair 50. The base unit, once activated supplies power to the aim LED 25, to laser activation light is press, causing the high intensity LED 24 to illuminate and then the laser to produce light for a predetermined time period, of sufficient duration to cause damage to the papilla of the hair follicle at hair 50, but not of sufficient duration to cause burning of the skin. Control at base unit 112 prevents firing of the laser again for a period sufficient to allow cooling of the user's skin.

Modifications, alternatives, variants, additions and simplifications to the embodiments described above, and illustrated, will be readily apparent to the skilled addressee. In particular, not all the safety features mentioned above need be used together, or the features mentioned could be combined.

## Claims

1. A laser hair removal device (10, 110) for being directed towards and/or applied to the skin of a user to expose hair follicles to laser radiation for hair removal, said device comprising:
a body (14).
a hair removal laser (18) housed in said body and capable of emitting radiation along a main axis at a wavelength suitable for hair removal,
a further light emitting element (24) arranged to emit high intensity radiation (21) selected to stimulate an eye blinking reflex and non-injurious to the eye, and
a control circuit which is configured to, at or following power up of the device, and prior to actuation of said hair removal laser, turn on said further light emitting element to emit a beam of radiation,
**characterised in that**, when turned on, said further light emitting element projects a high intensity beam of visible light along an axis in generally the same direction as said main axis thereby to provide a warning of the projected axis of the radiation from said hair removal laser and to deter a user from aiming the device towards the eye.

2. A laser hair removal device as claimed in claim 1 wherein the high intensity beam (21) is turned on as soon as the device is powered up.

3. A laser hair removal device as claimed in claim 1 or claim 2 wherein the hair removal laser (18) is capable of being fired on demand using a trigger (28).

4. A laser hair removal device as claimed in any wavelength preceding claim wherein the laser (18) is configured to generate a beam of pulsed laser radiation.

5. A laser hair removal device as claimed in any wavelength preceding claim wherein the light emitting element (24) is configured to emit light (21) at a wavelength of about 500-600nm to which the eye is particularly sensitive.

6. A laser hair removal device as claimed in claim 5 wherein said wavelength is about 555nm.

7. A laser hair removal device as claimed in claim 5 or 6 wherein the light emitting element (24) comprises an LED for providing light at the required wavelength.

8. A laser hair removal device (10,110) as claimed in any preceding claim wherein the body (14) of the device includes components mounted in an applicator wand which contains at least the majority of the control functionality required for operation.

9. A laser hair removal device as claimed in any of claims 1 to 7 wherein the device is in the form of a separate base unit (12,112) which contains the majority of the control circuitry with a separate wand (14) containing the laser unit and connected to the base unit by a flexible link (16).

10. A laser hair removal device as claimed in any of the preceding claims, which further includes a low intensity LED (25) for producing a low intensity narrow beam of light to enable a user to aim the hair removal laser.

## Patentansprüche

1. Vorrichtung zur Haarentfernung mittels Laser (10, 110), der gegen die Haut gerichtet und / oder auf der Haut eines Benutzers angewendet wird, um Haarfollikel der Laserstrahlung auszusetzen zum Zwecke der Haarentfernung, wobei die Vorrichtung die folgenden Teile aufweist: einen Körper (14), einen Haarentfernungslaser (18), der sich in dem Körper befindet und in der Lage ist, längs einer Hauptachse Strahlung zu emittieren, und zwar mit einer Wellenlänge, die sich für das Haarentfernen eignet, des weiteren ein weiteres Licht emittierendes Element (24), das so angeordnet ist, dass es Strahlung (21) hoher Intensität emittiert, die so gewählt ist, dass sie einen Augenzwinkerreflex stimuliert und das Auge nicht verletzt, und mit einer Steuerschaltung, die gebaut ist, damit sie die Vorrichtung mit Spannung versorgt oder unter Spannung hält und zur Betätigung des Haarentfernungslaser das weitere Licht emittierende Element einschaltet, um einen Strahl der Strahlung zu emittieren, **dadurch gekennzeichnet, dass** dann, wenn das weitere Licht emittierende Element eingeschaltet wird, es einen Strahl hoher Intensität sichtbaren Lichts längs einer Achse in etwa derselben Richtung, wie die Hauptachse verläuft, aussendet, um **dadurch** von dem Haarentfernungslaser eine Warnung betreffend die projizierte Strahlungsachse zu erzeugen und den Benutzer davon abzuhalten, mit der Vorrichtung auf das Auge zu zielen.

2. Vorrichtung zur Haarentfernung mittels Laser nach Anspruch 1, **dadurch gekennzeichnet, dass** der Strahl (21) hoher Intensität eingeschaltet wird, sobald die Vorrichtung unter Spannung bzw. Strom gesetzt wird.

3. Vorrichtung zur Haarentfernung mittels Laser nach Anspruch 1 oder Anspruch 2, **dadurch gekennzeichnet, dass** der Haarentfernungslaser (18) in der Lage ist, auf Anforderung eingeschaltet zu werden, und zwar durch Benutzung eines Triggers (28).

4. Vorrichtung zur Haarentfernung mittels Laser nach irgendeinem vorhergehenden Anspruch, **dadurch gekennzeichnet, dass** der Laser (18) gebaut ist, um einen Strahl einer gepulsten Laserstrahlung zu erzeugen.

5. Vorrichtung zur Haarentfernung mittels Laser nach irgendeinem vorhergehenden Anspruch, **dadurch gekennzeichnet, dass** das Licht emittierende Element (24) Licht (21) bei einer Wellenlänge von etwa 500 - 600 nm aussendet, für die das Auge besonders empfindlich ist.

6. Vorrichtung zur Haarentfernung mittels Laser nach Anspruch 5, **dadurch gekennzeichnet, dass** die Wellenlänge etwa 555 nm beträgt.

7. Vorrichtung zur Haarentfernung mittels Laser nach Anspruch 5 oder 6,
**dadurch gekennzeichnet, dass** das Licht emittierende Element (24) eine LED ist, um Licht mit der gewünschten Wellenlänge zu erzeugen.

8. Vorrichtung zur Haarentfernung mittels Laser (10, 100) nach irgendeinem vorhergehenden Anspruch, **dadurch gekennzeichnet, dass** der Körper (14) der Vorrichtung Komponenten aufweist, die in einer Applikationswand befestigt sind, welche wenigstens den Hauptteil der Steuerfunktion enthält, die für den Betrieb erforderlich ist.

9. Vorrichtung zur Haarentfernung mittels Laser nach einem der Ansprüche 1 bis 7, **dadurch gekennzeichnet, dass** die Vorrichtung in Form einer separaten Basiseinheit (12, 112) vorhanden ist, die den Hauptteil der Steuerschaltung mit einer separaten Wand (14) enthält, in der sich die Lasereinheit befindet, und die mit der Basiseinheit durch ein flexibles Gelenk (16) verbunden ist.

10. Vorrichtung zur Haarentfernung mittels Laser nach irgendeinem der vorhergehenden Ansprüche, **dadurch gekennzeichnet, dass** die Vorrichtung ferner eine LED (25) geringer Intensität aufweist, die zur Erzeugung eines Lichtstrahls niedriger Intensität dient, um einem Benutzer zu ermöglichen, mit der Vorrichtung zur Haarentfernung mittels Laser zu zielen.

## Revendications

1. - Dispositif d'épilation au laser (10, 110) destiné à être dirigé vers et/ou appliqué sur la peau d'un utilisateur pour exposer des follicules pileux à un rayonnement laser pour une épilation, ledit dispositif çomprenant :
un corps (14),
un laser d'épilation (18) reçu dans ledit corps et capable d'émettre un rayonnement le long d'un axe principal à une longueur d'onde appropriée pour l'épilation,
un élément supplémentaire d'émission de lumière (24) agencé pour émettre un rayonnement de haute intensité (21) sélectionné pour stimuler un réflexe de clignement de l'oeil et non nocif pour l'oeil, et
un circuit de commande qui est corifiguré pour, à ou suite à la mise en marche du dispositif, et avant l'actionnement dudit laser d'épilation, allumer ledit élément supplémentaire d'émission de lumière pour émettre un faisceau de rayonnement,
**caractérisé par le fait que**, lorsqu'il est allumé, ledit élément supplémentaire d'émission de lumière projette un faisceau de lumière visible de haute intensité le long d'un axe dans globalement la même direction que ledit axe principal, pour produire ainsi un avertissement de l'axe projeté du rayonnement à partir dudit laser d'épilation et dissuader ainsi un utilisateur d'orienter le dispositif vers l'oeil.

2. - Dispositif d'épilation au laser selon la revendication 1, dans lequel le faisceau de haute intensité (21) est allumé dès que le dispositif est mis en marche.

3. - Dispositif d'épilation au laser selon l'une des revendications 1 ou 2, dans lequel le laser d'épilation (18) est capable d'être démarré sur demande à l'aide d'un élément de déclenchement (28).

4. - Dispositif d'épilation au laser selon l'une quelconque des revendications précédentes, dans lequel le laser (18) est configuré pour générer une faisceau de rayonnement laser pulsé.

5. - Dispositif d'épilation au laser selon l'une quelconque des revendications précédentes, dans lequel l'élément d'émission de lumière (24) est configuré pour émettre une lumière (21) à une longueur d'onde d'environ 500-600 nm, à laquelle l'oeil est particulièrement sensible.

6. - Dispositif d'épilation au laser selon la revendication 5, dans lequel ladite longueur d'onde est d'environ 555 nm.

7. - Dispositif d'épilation au laser selon l'une des revendications 5 ou 6, dans lequel l'élément d'émission de lumière (24) comprend une diode électroluminescente destinée à produire une lumière à la longueur d'onde requise.

8. - Dispositif d'épilation au laser (10, 110) selon l'une quelconque des revendications précédentes, dans lequel le corps (14) du dispositif comprend des composants montés dans une crayon applicateur qui contient au moins la majorité de la fonctionnalité de commande requise pour le fonctionnement.

9. - Dispositif d'épilation au laser selon l'une quelconque des revendications 1 à 7, dans lequel le dispositif se présente sous la forme d'une unité de base séparée (12, 112) qui contient la majorité des circuits de commande, avec un crayon séparé (14) contenant l'unité laser et relié à l'unité de base par une liaison souple (16).

10. - Dispositif d'épilation'au laser selon l'une quelconque des revendications précédentes, lequel dispositif comprend en outre une diode électroluminescente de faible intensité (25) destinée à produire un faisceau de lumière étroit de faible intensité pour permettre à un utilisateur d'orienter le laser d'épilation.
